Europäisches Patentamt

European Patent Office

Office européen des brevets

(11) Veröffentlichungsnummer: **0 156 961 B1**

## (12) EUROPÄISCHE PATENTSCHRIFT

(45) Veröffentlichungstag der Patentschrift: 24.04.91

(51) Int. Cl.⁵: **A61K 37/14**, A61K 35/14, C07K 3/26

(21) Anmeldenummer: 84113547.8

(22) Anmeldetag: 09.11.84

Die Akte enthält technische Angaben, die nach dem Eingang der Anmeldung eingereicht wurden und die nicht in dieser Patentschrift enthalten sind.

(54) **Verfahren zur Herstellung hochgereinigter, stromafreier, hepatitissicherer Human- und Tierhämoglobinlösungen.**

(30) Priorität: 31.03.84 DE 3412144

(43) Veröffentlichungstag der Anmeldung:
09.10.85 Patentblatt 85/41

(45) Bekanntmachung des Hinweises auf die Patenterteilung:
24.04.91 Patentblatt 91/17

(84) Benannte Vertragsstaaten:
AT BE CH DE FR GB IT LI LU NL SE

(56) Entgegenhaltungen:
EP-A- 0 071 888
US-A- 3 991 181
US-A- 4 001 401
US-A- 4 401 652

(73) Patentinhaber: **Biotest Pharma GmbH**
**Flughafenstrasse 4**
**W-6000 Frankfurt 71(DE)**

(72) Erfinder: **Kothe, Norbert, Dipl.Chem.Dr.**
**Friedrich Ebert-Str. 21**
**W-6242 Kronberg(DE)**
Erfinder: **Eichentopf, Bertram, Dipl.Pharm.Dr.**
**Im Lauer 7**
**W-6232 Bad Soden(DE)**

(74) Vertreter: **Wolff, Hans Joachim, Dr.jur.**
**Dipl.-Chem. et al**
**Beil, Wolff & Beil Rechtsanwälte Postfach 80**
**01 40 Adelonstrasse 58**
**W-6230 Frankfurt am Main 80(DE)**

## Beschreibung

Die Erfindung betrifft ein Verfahren zur Herstellung hochgereinigter, stromafreier, hepatitissicherer Human- und Tierhämoglobinlösungen nach dem Obergriff des Anspruchs 1.

Hämoglobinlösungen sind in der Lage, unabhängig von Erythrozyten in vivo Sauerstoff zu transportieren. Für die Verträglichkeit solcher Lösungen ist es jedoch nötig, Plasma und Zellbestandteile des Blutes, aus dem das Hämoglobin durch Hämolyse freigesetzt wird, möglichst vollständig zu entfernen (Plasmaproteine, Stroma, etc.).

Zur Herstellung von Hämoglobinlösungen werden verschiedene Verfahren angewandt.

In der ersten Stufe erfolgt bei allen Verfahren eine Waschung der Erythrozyten, um die Plasmaproteine zu entfernen.
In den meisten der bisher bekannten Verfahren werden die Erythrozyten mit isotonen Elektrolytlösungen versetzt und durch Zentrifugation abgetrennt, wie beispielsweise gemäß der DE-PS 22 48 475.

Gemäß US-ps 3 991 181 werden zuerst die Erythrozyten durch Zentrifugation abgetrennt und anschließend mit isotonen Elektrolytlösungen versetzt.

In der DE-OS 31 30 770 wird ein Verfahren beschrieben, das auch für die großtechnische Herstellung geeignet ist und eine Sedimentation zur Waschung der Erythrozyten beinhaltet.

Gemeinsame nächste Stufe aller Herstellungsverfahren ist eine Hämolyse der Erythrozyten mit nachfolgender Abtrennung der Stromabestandteile, die entweder durch Filtration, Zentrifugation, Umkristallisation des Hämoglobins oder einer Kombination dieser Methoden erfolgen kann (DE-OS 31 30 770, US-PS 3 991 181, De Venuto et al. "Appraisal of Hemoglobin Solution as a Blood Substitute", Surgery, Gynecology & Obstetrics, Sept. 1979, Bd. 149, S. 417-436, F. De Venute et al "Characteristics of stroma free hemoglobin prepared by crystallization", J. Lab. Clin. Med., März 1977, S. 509-516; Mario Feola et al "Development of a bovine stroma-free hemoglobin solution as a blood substitute", Surgery, Gynecology & Obstetrics, 157 (5), November 1983, Seiten 399-408).

Dieser möglichst vollständigen Stromaabtrennung kommt eine besondere Bedeutung zu, da Reststromalipide unter Umständen die Blutgerinnung beeinflussen und Nierenschäden hervorrufen können.

Alle bisher zur Herstellung von Hämoglobinlösungen angewandten Verfahren weisen noch entscheidende Nachteile auf. Die Waschung der Erythrozyten in der bisher durchgeführten Weise führt zu Produkten, die noch Plasmaproteine enthalten. Eine vollständige Entfernung der Plasmaproteine durch wiederholte Waschung der Erythrozyten oder Umkristallisation des Hämoglobins führt zu erheblichen Ausbeuteverlusten und ist im großtechnischen Maßstab unwirtschaftlich.

Aus diesem Grunde war es auch bisher mit herkömmlichen Verfahren nicht möglich, in der Humanmedizin möglicherweise anzuwendende Hämoglobinlösungen aus Tierblut herzustellen, da die in den Lösungen vorhandenen Restproteine bei Anwendung am Menschen zu Immunreaktionen führen können.

Der Erfindung liegt die Aufgabe zugrunde, ein Verfahren bereitzustellen, das die Herstellung hochgereinigter Hämoglobinlösungen für die Humantherapie, die frei von Plasmaproteinen und Reststromalipiden sind, nicht nur aus Humanblut, sondern auch aus Tierblut in so großen Mengen erlaubt, wie sie für eine klinische Anwendung benötigt werden.

Diese Aufgabe wird erfindungsgemäß dadurch gelöst, daß man

a) das Waschen der Erythrozyten durchführt durch Umpumpen im Kreislauf über eine zu überströmende Filtereinheit einer Porengröße zwischen 0,1 und 1,2 μm, wobei verbrauchte Waschlösung als Filtrat kontinuierlich abgezogen und durch neue Waschlösung ergänzt wird,

b) die gewaschenen und ankonzentrierten Erythrozyten vom Filter gewinnt und in an sich bekannter Weise hämolysiert,

c) das Hämolysat bei nahezu konstantem Volumen über eine zu überströmende Ultrafiltrationseinheit einer Durchlässigkeit von 80 000 bis 100 000 D im Kreislauf gegen Wasser pumpt und

d) die in Stufe c) als Ultrafiltrat anfallende verdünnte Hämoglobinlösung bei nahezu konstantem Volumen über eine zu überströmende Ultrafiltrationseinheit mit einer Durchlässigkeit von 10 000 bis 50 000 D im Kreislauf gegen Wasser pumpt und dabei diafiltriert, wobei vor und/oder nach der Diafiltration durch die gleiche Ultrafiltrationseinheit ohne Wassergegenstrom solange im Kreislauf gepumpt wird, bis das Hämoglobin auf den gewünschten Wert ankonzentriert ist.

Mit Hilfe der erfindungsgemäßen Kombination von Filtrationen mit Filtermembranen absteigender Porendurchmesser gelingt es, die Erythrozyten von Plasmaproteinen zu befreien, nach Lyse die Erythrozytenhüllen vollständig zu entfernen und das Hämoglobin von unerwünschten Begleitsubstanzen zu reinigen.

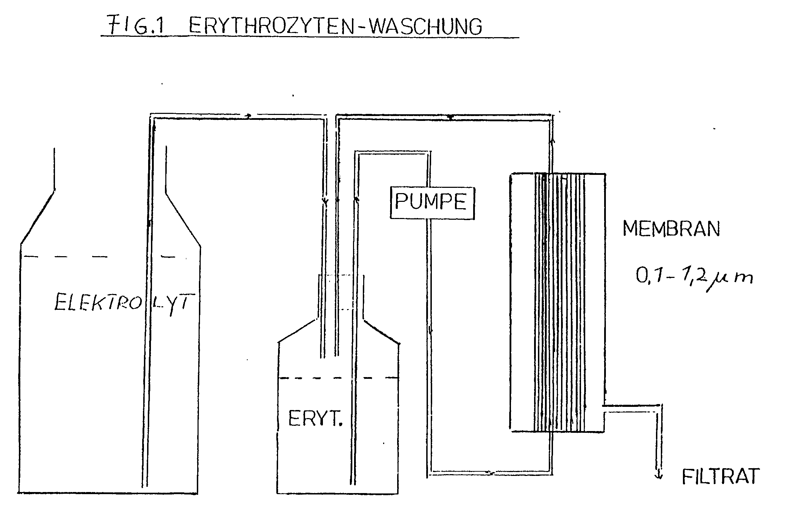

Die in Fig. 1 schematisch gezeigte erste Verfahrensstufe stellt die Erythrozyten-Waschung dar, bei der die Erythrozyten im Kreislauf über eine Filtereinheit gegen eine isotone Waschlösung gepumpt werden. Dabei lassen sich technische Pumpen und technische Filtrationsmodulen einsetzen, wie sie z.B. üblicherweise zur Rückgewinnung von Lackpigmenten verwendet werden.

Bekanntlich unterliegen Erythrozyten sehr leicht der Hämolyse, und der Fachmann ist stets bemüht, Erythrozyten so schonend wie möglich zu waschen, um Hämolyse zu vermeiden. Ganz besonders empfindlich gegenüber Hämolyse sind alte, überlagerte Erythrozyten-Konzentrate.

Es war deshalb völlig überraschend, daß bei einem Im-Kreislauf-Pumpen unter Verwendung technischer Pumpen über technische Filtermodule , was dem Fachmann nicht gerade als schonende Behandlung anmuten mußte, kaum Hämolyse erfolgte, was ganz besonders überraschend für den Einsatz überlagerter Erythrozyten-Konzentrate war.

Somit läßt sich das erfindungsgemäße Verfahren nicht nur bei frischem Vollblut tierischer und humaner Herkunft, sondern auch bei überlagerten Human-Erythrozyten-Konzentraten anwenden, ohne daß eine nennenswerte Hämolyse eintritt.

Mit Hilfe dieser ersten Verfahrensstufe gelingt es, in kurzer Zeit große Mengen Waschlösung mit Erythrozyten in Kontakt zu bringen und wieder aus dem Kreislauf zu entfernen, so daß eine intensive Waschung erfolgt.

Als Waschlösung eignen sich jegliche isotone Elektrolytlösungen sowie isotone Lösungen von Zucker oder Zuckeralkoholen. Eine bevorzugte Waschlösung ist isotone NaCl-Lösung.

Durch Vergrößerung der Filterflächen lassen sich beliebige Erythrozytenvolumina waschen, so daß dieses Verfahren für die großtechnische Herstellung von Hämoglobinlösungen geeignet ist.

Die verwendeten Filter können sowohl Flach- als auch Hohlfasermembranen enthalten. Hohlfasermembranen werden bevorzugt, weil sie mehr Fläche auf kleinem Raum aufweisen als Flachmembranen.

Die durchschnittliche Porengröße liegt zwischen 0,1 und 1,2 $\mu$m. Bei Unterschreitung der unteren Grenze könnten Plasmaproteine mit den Erythrozyten zurückgehalten werden, während bei Überschreiten der oberen Grenze sich die Hämolyserate drastisch erhöht. Vorzugsweise werden Membranen mit einer mittleren Porengröße von 0,4 bis 0,6 $\mu$m verwendet.

Der Filtrationsdruck sollte zweckmäßigerweise zwischen $5.10^3$ und $7.10^4$ Pa (50 - 700 mbar) liegen.

Die Kreislaufwäsche erfolgt so lange, bis keine Plasmaproteine mehr nachweisbar sind.

Nach Waschung der Erythrozyten in der beschriebenen Art und Weise erfolgt die Hämolyse durch Einbringen der ankonzentrierten Erythrozyten in das 2-3 fache Volumen Wasser.

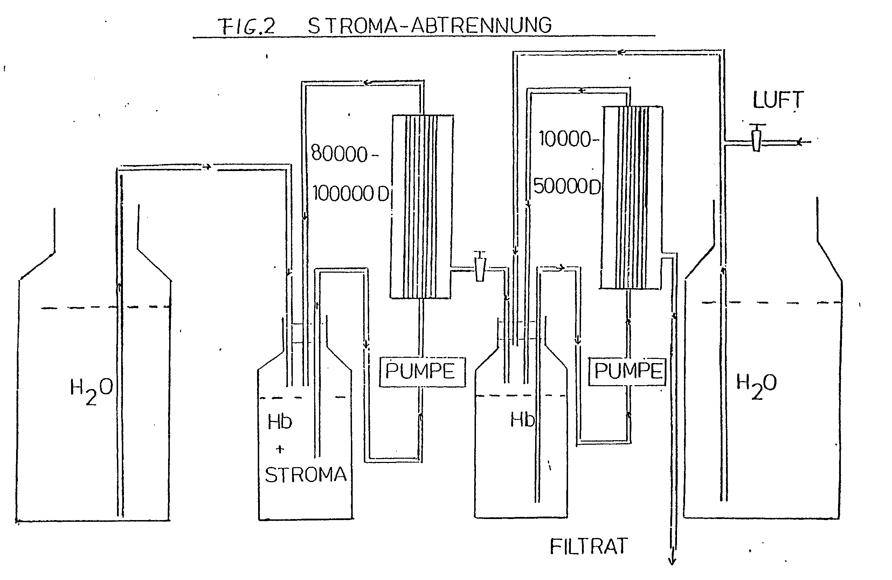

Die in Fig. 2 gezeigte zweite und dritte Verfahrensstufe stellt die Abtrennung des Stromas vom Hämoglobin dar, dabei wird unmittelbar anschließend an die Hämolyse das aus den Erythrozyten freigesetzte Hämoglobin in einer zweiten Filtrationssstufe von dem Erythrozytenstroma abgetrennt. Hierzu wird das Hämolysat intensiv gegen Wasser diafiltriert, wobei eine Ultrafiltrationseinheit mit einer Durchlässigkeit von 80 000 bis 100 000 D verwendet wird (Flachmembranen oder Hohlfasermembranen, vorzugsweise Hohlfasermembranen).

Dies bedeutet, daß das Hämolysat bei nahezu konstantem Volumen im Kreislauf über die Ultrafiltrationseinheit gepumpt wird, wobei das Hämoglobin mit einem Molekulargewicht von 64 500 zusammen mit allen Begleitstoffen, deren Molekulargewicht weniger als 80 000 bis 100 000 D beträgt, im Ultrafiltrat erscheint.

Vorteil dieses Verfahrens ist, daß nahezu das gesamte Hämoglobin aus dem vorliegenden Hämolysat gewonnen werden kann, im Gegensatz zu bisher bekannten Verfahren, bei denen ein Großteil des Hämoglobins zusammen mit dem Erythrozytenstroma verworfen wird.

Die in dieser zweiten Filtrationsstufe als Ultrafiltrat anfallende verdünnte Hämoglobinlösung wird in einer dritten Filtrationsstufe, parallel zur zweiten Filtration, ankonzentriert. Verwendet wird wiederum eine Ultrafiltrationseinheit, jedoch mit einer Durchlässigkeit von 10 000 bis 50 000 D, so daß das Hämoglobin zurückgehalten wird und damit sowohl ankonzentriert als auch bereits teilweise von Begleitsubstanzen mit Molekulargewichten unter der genannten Durchlässigkeit der eingesetzten Membran befreit wird. Sobald das Hämoglobin auf einen erwünschten Wert ankonzentriert ist, wird zur vollständigen Abreicherung kleinmolekularer Begleitstoffe erneut gegen Wasser diafiltriert.

Die Diafiltration kann jedoch praktisch an jeder Stelle der dritten Filtrationsstufe erfolgen. Es kann beispielsweise erst diafiltriert und dann konzentriert werden oder umgekehrt. Nachdem jedoch zum Zwecke des Diafiltrierens das Volumen der Hämoglobinlösung weder zu groß noch die Lösung zu konzentriert sein sollte, erfolgt die Diafiltration zweckmäßigerweise etwa in der Mitte der dritten Stufe, d.h. es wird erst auf einen gewissen Wert ankonzentriert, dann diafiltriert und danach auf die gewünschte Endkonzentration eingestellt.

Der Filtrationsdruck in der zweiten und dritten Filtrationsstufe erfährt seine Begrenzung lediglich durch den Maximaldruck,der für die jeweiligen entsprechenden Filtermodule zulässig ist. Dieser ist vom Hersteller auf den Modulen angegeben.

Es resultiert ein hochgereinigtes Hämoglobin gewünschter Konzentration, das nach üblichen Verfahren zu modifizierten Hämoglobinlösungen verarbeitet werden kann, wie beispielsweise in DE-PS 24 49885 , 26 17 822 und 27 14 252 beschrieben, wobei sich vorstehende Literaturangaben nur auf Modifizieren und nicht auf Reinigen beziehen sollen.

Bei den Filtrationen in allen drei Filtrationsstufen handelt es sich um sogenannte tangential überströmende Filtrationen. Bei einer üblichen Filtration würden bei dem zu filtrierenden Material sehr schnell die Poren verstopfen.

Als Wasser wird zweckmäßigerweise entsalztes oder destilliertes Wasser genommen.

Das in der bereits vorstehend genannten Druckschrift Mario Feola et al. beschriebene Verfahren zur Herstellung von Hämoglobinlösungen beinhaltet zwar auch zwei Stufen, in denen das Hämolysat hintereinander durch Filter mit einer Durchlässigkeit von 100 000 D und 30 000 D geschickt wird. Die vorangehende Erythrozyten-Waschung erfolgt in diesem Verfahren jedoch auf übliche Weise mit einer Elektrolytlösung, wobei keine vollständige Abtrennung des Plasmaproteins erfolgt. Ferner wird das Ultrafiltrat aus der letzten 30 000 D-Filtration gegen einen Elektrolyten dialysiert, was zur Folge hat, daß die daraus resultierende Hämoglobinlösung einen hohen Elektrolytgehalt aufweist, was für spezielle Verwendungszwecke, z.B. Perfusion der Herzkranzgefäße bei Operationen am offenen Herzen (Kardioplegie) von Nachteil ist. Mit Hilfe des erfindungsgemäßen Verfahrens, bei dem in der zweiten und dritten Stufe gegen Wasser diafiltriert wird, wird dagegen ein praktisch elektrolytfreies Produkt erhalten.

Schließlich ist den beiden Ultrafiltrationen nach erfolgter Hämolyse eine Filtration durch ein Filter mit einer Porengröße von 0,5 μm vorgeschaltet, weil der Fachmann annehmen mußte, daß bei der Filtration eines Hämolysats, das noch alle groben Zellbestandteile enthält, unmittelbar durch ein 100 000 D Filter, dieses Filter sehr schnell verstopfen würde.

Überraschenderweise fand man jedoch, daß, wenn man gemäß der Erfindung das Hämolysat über ein 100 000 D Filter im Kreislauf gegen Wasser pumpt, keine Filterverstopfung stattfindet und eine vorgeschaltete Filtrationsstufe für das Hämolysat durch ein gröberes Filter entfallen kann.

Die Vorteile des erfindungsgemäßen Kombinationsverfahrens liegen zum einen in der technisch einfachen Realisierbarkeit, verbunden mit einem beträchtlichen Zeitgewinn und einer erhöhten Hämoglobinausbeute gegenüber bisher bebeschriebenen Verfahren; zum anderen ermöglicht die hohe Reinheit des so gewonnenen Hämoglobins möglicherweise den Einsatz von Tierblut zur Herstellung modifizierter Hämoglobinlösungen für die Humantherapie. Darüberhinaus ist das Produkt praktisch frei von Elektrolyten,und das Verfahren führt zu einer drastischen Reduzierung eventuell im Ausgangsmaterial vorhandener Bakterien oder Viren, so daß auch die aus bisherigen Verfahren bekannte chemische Sterilisation, beispielsweise mit β-Propiolacton (z.B. DE-PS 22 48 475, DE-OS 31 30 770) entfallen kann. Für besondere Fälle läßt sich jedoch gegebenenfalls eine β-Propiolactonbehandlung auch beim erfindungsgemäßen Verfahren während der Erythrozyten-Waschung durchführen.

Nachstehende Beispiele dienen der weiteren Erläuterung der Erfindung.

Beispiel 1

5 l frisches, stabilisiertes Rinderblut wurde über ein Blutfilter in ein 5 l-Gefäß filtriert. Mit Hilfe einer Blutpumpe wurde das Blut im Kreislauf über zwei Hohlfaserfiltrationspatronen der Porenweite 0,4 μm und einer Gesamtfläche von 1 m² gepumpt. Das aus dem Kreislauf abfiltrierte Volumen wurde kontinuierlich durch 0,9%ige NaCl-Lösung ersetzt. Zur nahezu vollständigen Entfernung der Plasmaproteine wurden 50 l (10-faches Ausgangsvolumen) NaCl-Lösung zur Waschung eingesetzt. Der mittlere Filtrationsdruck betrug $2,66.10^4$ Pa (266 mbar). Die Waschung war in 3 Stunden beendet. Danach wurden die Erythrozyten auf 2,5 l ankonzentriert. Dieses Erythrozytenkonzentrat wurde in 5 l destilliertes Wasser eingerührt, wobei Hämolyse eintrat. Zur Stromaentfernung wurde das Hämolysat im Kreislauf über 2 Hohlfaserpatronen (1,8 m²) der Durchlässigkeit 100 000 D gepumpt. Das Hämoglobin (MG 64 500) passierte die Membran, während die Stromabestandteile und höhermolekularen Substanzen zurückgehalten wurden. Das Kreislaufvolumen wurde durch kontinuierliche Zugabe von destilliertem Wasser konstant gehalten. In 30 l Ultrafiltrat waren ca. 95 % des gesamten Hämoglobins enthalten. Sobald 5 l Ultrafiltrat vorlagen, wurde unter Konstanthaltung des Volumens das Hämoglobin in einem weiteren Kreislauf, in dem sich zwei Hohlfaserpatronen der Durchlässigkeit 10 000 D (1,8 m²) befanden, ankonzentriert. Sobald die Stromaabtrennung beendet war und 95 % des Gesamthämoglobins in dem Volumen von 5 l vorlagen, wurde zur Entfernung niedermolekularer Bestandteile über die gleiche Ultrafiltrationseinheit mit einer Durchlässigkeit von 10 000 D gegen 25 l destillierten Wassers diafiltriert. Im letzten Schritt wurde im selben Kreislauf auf 22 % Hämoglobin ankonzentriert. Anschließend wurde die Lösung über ein 0,2 μm Filter sterilfiltriert.

Ausbeute:     80 %.

Beispiel 2

Die vollständige Entfernung der Plasmaproteine aus der Hämoglobinlösung ließ sich anhand von Immunelektrophoresen und im Tierexperiment zeigen. Hierzu wurden Meerschweinchen mit einer nach Beispiel 1 hergestellten Hämoglobinlösung unter Zuhilfenahme von Freund'schem Adjuvans immunisiert. Im Plasma der Tiere ließen sich keine Antikörper gegen Humanplasmaproteine bzw. gegen tierische Plasmaproteine nachweisen

Beispiel 3

Die drastische Verringerung vorhandener Erreger im Ausgangsblut durch das erfindungsgemäße Verfahren ließ sich ebenfalls im Experiment zeigen. Setzte man dem zur Herstellung der Hämoglobinlösung verwendeten Blut entweder $\phi$ X 174 Phagen mit einem Durchmesser von ca.25nm oder K' Phagen mit einem Durchmesser von 200 nm in einer Konzentration von $10^7$/ml zu, so ließ sich zeigen, daß durch Waschung der Erythrozyten mit dem 10-fachen Waschlösungsvolumen in der ersten Filtrationsstufe eine Abreicherung um $10^3$ - $10^4$ Phagen erfolgt.

In der nächsten Filtrationsstufe, bei dem das freigesetzte Hämoglobin durch eine Ultrafiltrationsmembran mit einer Durchlässigkeit von 100 000 D abfiltriert wurde, wurden weitere $10^3$/ml Phagen durch die Membran zurückgehalten, da sie aufgrund ihres Durchmessers die Membran nicht passieren konnten. Insgesamt wurden damit die zugesetzten Phagen um ca. $10^6$/ml, also auf 10/ml, verringert. Dieser Sterilisationseffekt ist deshalb von besonderer Bedeutung, weil nach üblichen Blutbankkriterien geprüftes Transfusionsblut nach wie vor eine Hepatitis übertragen kann. Wie an früherer Stelle bereits ausgeführt, war bei bisherigen Verfahren deshalb ein chemisches Sterilisationsverfahren erforderlich. Durch das hier beschriebene Filtrationsverfahren kann eine solche chemische Sterilisation entfallen.

Ansprüche

1. Verfahren zur Herstellung hochgereinigter, stromafreier heptatitissicherer Human- und Tierhämoglobinlösungen, wobei man das Erythrozyten enthaltende Ausgangsmaterial mit einer isotonen Waschlösung wäscht, die gewaschenen Erythrozyten gewinnt und hämolysiert und hintereinander eine Filtration des Hämolysats mit Hilfe einer Ultrafiltrationseinheit mit einer Durchlässigkeit von 100 000 D und einer Durchässigkeit von 30 000 D durchführt, **dadurch gekennzeichnet,** daß man
a) das Waschen der Erythrozyten durchführt durch Umpumpen im Kreislauf über eine zu überströmende Filtereinheit einer Porengröße zwischen 0,1 und 1,2 $\mu$m, wobei verbrauchte Waschlösung als Filtrat kontinuierlich abgezogen und durch neue Waschlösung ergänzt wird,
b) die gewaschenen und ankonzentrierten Erythrozyten vom Filter gewinnt und in an sich bekannter Weise hämolysiert,
c) das Hämolysat bei nahezu konstantem Volumen über eine zu überströmende Ultrafiltrationseinheit einer Durchlässigkeit von 80 000 bis 100 000 D im Kreislauf gegen Wasser pumpt und
d) die in Stufe c) als Ultrafiltrat anfallende verdünnte Hämoglobinlösung bei nahezu konstantem Volumen über eine zu überströmende Ultrafiltrationseinheit mit einer Durchlässigkeit von 10 000 bis 50 000 D im Kreislauf gegen Wasser pumpt und dabei diafiltriert, wobei vor und/oder nach der Diafiltration durch die gleiche Ultrafiltrationseinheit ohne Wassergegenstrom solange im Kreislauf gepumpt wird, bis das Hämoglobin auf den gewünschten Wert ankonzentriert ist.

2. Verfahren mach Anspruch 1, dadurch gekennzeichnet, daß man in Stufe d) zuerst im Kreislauf pumpt, bis das Hämoglobin auf den gewünschten Wert ankonzentriert ist, danach durch die gleiche Ultrafiltrationseinheit im Kreislauf gegen Wasser diafiltriert und dann das Hämoglobin auf die gewünschte Endkonzentration einstellt.

3. Verfahren nach Anspruch 1 oder 2, dadurch gekennzeichnet, daß in Stufe a) bei einem Filtrationsdruck von $5.10^3$ bis $7.10^4$ Pa (50 bis 700 mbar) gearbeitet wird.

4. Verfahren nach einem der vorstehenden Ansprüche, dadurch gekennzeichnet, daß als Waschlösung in Stufe a) isotone NaCl-Lösung verwendet wird.

5. Verfahren nach einem der vorstehenden Ansprüche, dadurch gekennzeichnet, daß man in Stufe a) eine Filtrationseinheit mit einer Porengröße von 0,4 bis 0,6 $\mu$m verwendet.

6. Verfahren nach einem der vorstehenden Ansprüche, dadurch gekennzeichnet, daß man in den Stufen a), c) und d) als Filtrations- bzw. Ultrafiltrationseinheit eine Hohlfasermembran verwendet.

Claims

1. A process for the preparation of highly purified, stroma free, heptatitis-proof human and animal haemoglobin solutions, in which the starting material containing erythrocytes is washed with an isotonic washing solution and the washed erythrocytes are recovered and haemolyzed and the haemolysate is filtered successively with an ultrafiltration unit having a permeability of 100,000 D and a permeability of 30,000 D, characterised in that

a) washing of the erythrocytes is carried out by pump circulating them over a filter unit having a pore size of from 0.1 to 1.2 μm over which fluid is required to flow, spent washing solution being continuously withdrawn as filtrate and replaced by fresh washing solution,

b) the washed and partly concentrated erythrocytes are recovered from the filter and haemolyzed in known manner,

c) the haemolysate is pumped at almost constant volume in a circulation against water over an ultrafiltration unit which has a permeability of from 80,000 to 100,000 D, over which the fluid is required to flow, and

d) the diluted haemoglobin solution obtained as ultrafiltrate in stage c) is pumped at almost constant volume in a circulation against water over an ultrafiltration unit having a permeability of from 10,000 to 50,000 D over which the fluid is required to flow and is thus diafiltered, and pumping in circulation is continued through the same ultrafiltration unit without a counterflow of water, either before and/or after diafiltration, until the haemoglobin has been concentrated to the desired value.

2. A process according to Claim 1, characterised in that in stage d), pumping is first carried out in circulation until the haemoglobin has been concentrated to the desired value and diafiltration is thereafter carried out in circulation against water through the same ultrafiltration unit and the haemoglobin is then adjusted to the desired final concentration.

3. A process according to Claim 1 or Claim 2, characterised in that stage a) is carried out under a filtration pressure of from 5x10³ to 7x10 Pa (50 to 700 mbar).

4. A process according to one of the preceding claims, characterised in that the washing solution used in stage a) is isotonic NaCl solution.

5. A process according to one of the preceding claims, characterised in that a filtration unit having a pore size of from 0.4 to 0.6 μm is used in stage a).

6. A process according to one of the preceding claims, characterised in that the filtration or ultrafiltration unit used in stages a), c) and d) is a hollow fibre membrane.

**Revendications**

1. Procédé de préparation de solutions d'hémoglobine humaine et animale hautement purifiées exemptes de stroma et résistantes à l'hépatite dans lequel
on lave le produit de départ contenant les érythrocytes avec une solution de lavage isotonique,
on recueille les érythrocytes lavées et on les hémolyse et on effectue successivement une filtration de l'hémolysat à l'aide d'une unité d'ultrafiltration d'une perméabilité de 100 000 D et d'une perméabilité de 30 000 D caractérisé en ce que

a) on effectue le lavage des érythrocytes par un transvasement avec une pompe selon un parcours fermé en passant par une unité de filtration à traverser ayant une taille de pores comprise ente 0,1 et 1,2 μm, dans lequel la solution de lavage usée est continuellement retirée sous forme de filtrat et est remplacée par une nouvelle solution d'eau de lavage,

b) on recueille au filtre les érythrocytes lavées et concentrées et on les hémolyse de façon connue,

c) on pompe contre l'eau dans la circulation l'hémolysat à volume presque constant par l'intermédiaire d'une unité d'ultrafiltration à traverser ayant une perméabilité de 80 000 à 10 000 D et

d) on pompe contre l'eau dans la circulation la solution d'hémoglobine diluée apparue dans l'étape c) sous forme d'ultrafiltrat à volume presque constant à travers une unité d'ultrafiltration à traverser ayant une perméabilité de 10 000 à 50 000 D et on diafiltre, où avant et/ou après la diafiltration on pompe dans la circulation à travers la même unité d'ultrafiltration sans contre courant d'eau jusqu'à ce que l'hémoglobine soit concentrée à la valeur désirée.

2. Procédé selon la revendication 1, caractérisé en ce que dans l'étape d) on pompe tout d'abord dans la circulation jusqu'à ce que l'hémoglobine soit concentrée à la valeur désirée, puis on diafiltre contre l'eau dans la circulation à travers la même unité d'ultrafiltration et on règle l'hémoglobine à la concentration finale désirée.

3. Procédé selon la revendication 1 ou 2, caractérisé en ce que dans l'étape a) on travaille à une pression de filtration de $5 \times 10^3$ à $7 \times 10^4$ Pa (50 à 700 mbar).

4. Procédé selon l'une des revendications précédentes, caractérisé en ce qu'on utilise comme solution de lavage dans l'étape a) une solution de NaCl isotonique.

5. Procédé selon l'une des revendications précédentes, caractérisé en ce que dans l'étape a) on utilise une unité d'ultrafiltration ayant une taille de pores de 0,4 à 0,6 $\mu$m.

6. Procédé selon l'une des revendications précédentes, caractérisé en ce que dans les étapes a), b) et c) et d) on utilise comme unité de filtration ou selon les cas d'ultrafiltration une membrane à fibres creuses.

## FIG.1   ERYTHROZYTEN-WASCHUNG

ELEKTROLYT

ERYT.

PUMPE

MEMBRAN

$0,1 - 1,2 \mu m$

FILTRAT

EP 0 156 961 B1

FIG.2  STROMA-ABTRENNUNG

LUFT

80000 - 100000 D

10000 - 50000 D

$H_2O$

Hb + STROMA

PUMPE

Hb

PUMPE

$H_2O$

FILTRAT

EP 0 156 961 B1